# EUROPEAN PATENT SPECIFICATION

(11) **EP 2 771 312 B1**
(45) Date of publication and mention of the grant of the patent: **31.05.2017**
(21) Application number: 12799022.4
(22) Date of filing: 29.11.2012
(51) Int. Cl.: C07C 233/18, C07C 275/02

(54) **AGOMELATINE-UREA COMPLEX AND CRYSTALLINE FORMS THEREOF**
AGOMELATIN-HARNSTOFF-KOMPLEX UND KRISTALLINE FORMEN DAVON
COMPLEXE AGOMÉLATINE-URÉE ET FORMES CRISTALLINES DE CELUI-CI

(30) Priority: 30.11.2011 US 201161565224 P
(43) Date of publication of application: 03.09.2014
(73) Proprietor: ratiopharm GmbH, 89079 Ulm (DE)
(72) Inventor: HAFERKAMP, Sven, 44789 Bochum (DE); BOESE, Roland, 45326 Essen (DE)
(74) Representative: D Young & Co LLP
(86) International application number: PCT/US2012/067098
(87) International publication number: WO 2013/082302

(56) References cited:
- DE-A1-102007 030 695
- SAI-LI ZHENG ET AL: "Structures of Polymorphic Agomelatine and Its Cocrystals with Acetic Acid and Ethylene Glycol", CRYSTAL GROWTH & DESIGN, ACS, WASHINGTON, DC, US, vol. 11, 2 February 2011 (2011-02-02), pages 466-471, XP002658583, ISSN: 1528-7483, DOI: 10.1021/CG101234P [retrieved on 2011-01-06]
- YAN YAN ET AL: "Improving the Solubility of Agomelatine via Cocrystals", CRYSTAL GROWTH & DESIGN, vol. 12, no. 5, 2 May 2012 (2012-05-02), pages 2226-2233, XP055028201, ISSN: 1528-7483, DOI: 10.1021/cg201423q

## Description

### FIELD OF INVENTION

The present invention concerns an Agomelatine-urea complex, solid state forms of said Agomelatine-urea complex, processes for their preparation, and pharmaceutical compositions comprising an Agomelatine-urea complex or a solid state form thereof.

### BACKGROUND OF THE INVENTION

Agomelatine, N-[2-(7-methoxynaphthalen-1-yl)ethyl]acetamide, has the structural formula: 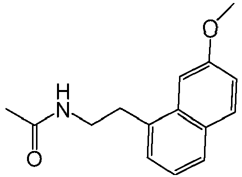 Agomelatine is marketed under the tradenames Valdoxan^{®} and Thymanax^{®}. Agomelatine is an antidepressant developed for the treatment of major depressive disorder. Agomelatine has been reported to have a reduced level of sexual side effects as well as discontinuation effects, as compared to some other antidepressants. Agomelatine may also have positive effects on sleep.

Agomelatine, its preparation and its use are disclosed in U.S. patent no. 5,225,442. European patent no. EP1564202 discloses Form II of Agomelatine. European patent nos. EP1752445, EP2008993 and EP2210872 disclose Form III of Agomelatine. European patent nos. EP1752444 and EP2008994 disclose Form IV of Agomelatine. European patent nos. EP1752443 and EP2277857 disclose Form V of Agomelatine. European patent no. EP2058296 and Chinese patent no. CN101585779 disclose Form VI of Agomelatine. Chinese patent no. CN101955440 discloses Form X of Agomelatine. Chinese patent no. CN101723844 discloses Form B of Agomelatine. DE102007030695 discloses cocrystals of urea and amide and/or urea derivatives. Zheng, S.-L. et al, Crystal Growth & Design, 2011 (11), 466-471 discloses agomelatine cocrystals with acetic acid and ethylene glycol.

Polymorphism, the occurrence of different crystal forms, is a property of some molecules and molecular complexes. A single molecule, like Agomelatine, may give rise to a variety of polymorphs having distinct crystal structures and physical properties like melting point, thermal behaviors (e.g., measured by capillary melting point, thermogravimetric analysis (TGA), or differential scanning calorimetry (DSC), as well as content of solvent in the polymorphic form), powder x-ray diffraction pattern ("PXRD" or "powder XRD"), infrared absorption and Raman fingerprints, and solid state NMR spectrum. The differences in physical properties have been used to distinguish polymorphic forms. One or more of these techniques may thus be used to characterize a particular solid state form and to distinguish different polymorphic forms of a compound. These techniques may also be used to quantify the amount of one or more crystalline forms in a mixture. The differences in the physical properties of different polymorphic forms result from the orientation and intermolecular interactions of adjacent molecules or complexes in the bulk solid. Accordingly, polymorphs are distinct solids sharing the same molecular formula yet having distinct physico-chemical properties compared to other polymorphic forms of the same compound or complex.

A co-crystal is a molecular complex with a crystalline structure composed of at least two components, wherein the components may be atoms, ions or molecules. A co-crystal consists of two or more components that form a unique crystalline structure having unique properties. A co-crystal structure exhibits long-range order and the components interact via non-covalent interactions such as hydrogen bonding, ionic interactions, van der Waals interactions and n-interactions.

The discovery of new polymorphic forms and co-crystals of Agomelatine can provide new ways to improve the synthesis and the characteristics of Agomelatine as an active pharmaceutical ingredient.

### SUMMARY OF THE INVENTION

The present invention provides an Agomelatine-urea complex, isolated and/or solid state forms of the Agomelatine-urea complex, and pharmaceutical compositions comprising said Agomelatine-urea complex and at least one pharmaceutically acceptable excipient.

According to some embodiments of the present invention, the Agomelatine-urea complex comprises co-crystals of Agomelatine and urea. According to some sub-embodiments thereof, the Agomelatine-urea complex comprises co-crystals of Agomelatine and urea in which the Agomelatine and urea are in a stoichiometry of about 1:1, or exactly 1:1. In a more preferred embodiment, the agomelatine urea complex is an agomelatine urea co-crystal.

The invention further provides the use of the Agomelatine-urea complex, and the solid state forms of the Agomelatine-urea complex described below, in the manufacture of a pharmaceutical composition. The invention further provides the Agomelatine-urea complex, and the solid state forms of the Agomelatine-urea complex described below, for use in the treatment of depression. The invention also provides a pharmaceutical composition comprising an Agomelatine-urea complex, or a crystalline form thereof, and at least one pharmaceutically acceptable excipient for use in the treatment of depression.

### BRIEF DESCRIPTION OF THE FIGURES

Figure 1 shows a powder XRD pattern of crystalline Agomelatine-urea complex.

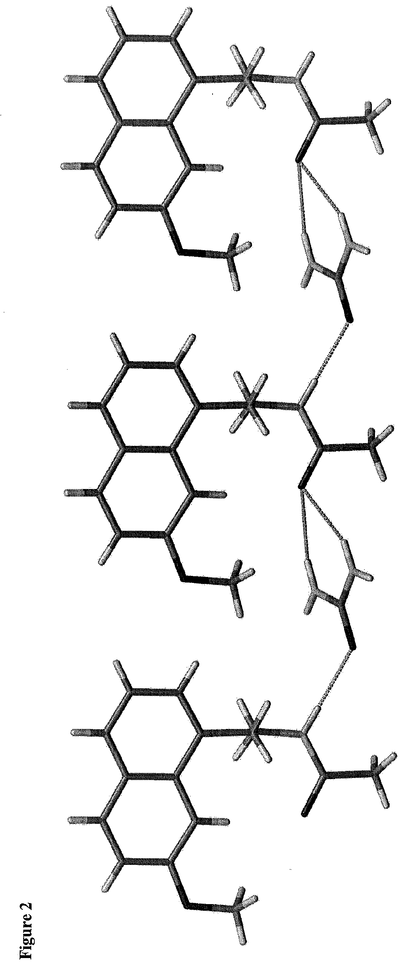

Figure 2 shows the interaction of Agomelatine and urea in crystalline Agomelatine-urea complex.
Figure 3 shows a ¹³C CPMAS NMR spectrum of crystalline Agomelatine-urea complex.

### DETAILED DESCRIPTION OF THE INVENTION

A crystal form may be referred to herein as being characterized by graphical data "as shown in," or "as depicted in," or "substantially as depicted in" a Figure. Such data include, for example, powder X-ray diffractograms and solid state NMR spectra. The graphical data potentially provides additional technical information to further define the respective solid state form which can not necessarily be described by reference to numerical values or peak positions. In any event, the skilled person will understand that such graphical representations of data may be subject to small variations, e.g., in peak relative intensities and peak positions due to factors such as variations in instrument response and variations in sample concentration and purity, which are well known to the skilled person. Nonetheless, the skilled person would readily be capable of comparing the graphical data in the Figure disclosed herein with graphical data generated for an unknown crystal form, and confirm whether the two sets of data are characterizing the same crystal form or two different crystal forms. The crystal form characterized by the graphical data "as shown in," or "as depicted in," or "substantially as depicted in" a Figure disclosed herein includes a crystal form characterized by graphical data with small variations, which are well known to the skilled person, in comparison to the graphical data in the Figure.

A crystal form (also referred to herein as a crystalline form or a polymorph) may be referred to herein as pure or polymorphically pure, or substantially free of any other crystalline or polymorphic forms. As used herein in this context, the expression "substantially free" will be understood to mean that the crystalline form contains 20% or less, 10% or less, 5% or less, 2% or less, or 1% or less of any other crystalline forms of the subject compound as measured, for example, by PXRD. Thus, crystalline forms of Agomelatine-urea complex described herein as substantially free of any other crystalline forms would be understood to contain greater than 80% (w/w), greater than 90% (w/w), greater than 95% (w/w), greater than 98% (w/w), or greater than 99% (w/w) of the subject crystalline form of Agomelatine-urea complex. Accordingly, in some embodiments of the invention, the described crystalline form may contain from 1% to 20% (w/w), from 5% to 20% (w/w), or from 5% to 10% (w/w) of one or more other crystal forms of the same complex.

A thing, *e.g.,* a reaction mixture, may be characterized herein as being at, or allowed to come to "room temperature" or "ambient temperature", often abbreviated "RT." This means that the temperature of the thing is close to, or the same as, that of the space, *e.g.,* the room or fume hood, in which the thing is located. Typically, room or ambient temperature is from about 15°C to about 30°C, or about 20°C to about 25°C, or about 25°C.

As used herein, the term "complex" refers to a molecular entity formed by association involving two or more component molecular entities (ionic or uncharged), or by association involving two or more chemical species. The bonding between the components is non-covalent and is normally weaker than covalent bonding. Accordingly, the Agomelatine-urea complex described herein is a molecular entity formed by the association between Agomelatine and urea. The Agomelatine-urea complex may in some embodiments exist as a solid state form that is referred to herein as a co-crystal form of an Agomelatine-urea complex, or as an Agomelatine-urea co-crystal, or as a crystalline Agomelatine-urea complex.

Co-crystals of Agomelatine are crystalline structures composed of Agomelatine and at least one other component. Agomelatine co-crystals form unique crystalline structures having unique properties. The intermolecular interactions in the co-crystals generate physical and chemical properties that differ from the properties of the individual components. Such properties include bioavailability, melting point, solubility, chemical stability, and mechanical properties. Agomelatine co-crystals can exhibit polymorphism.

All of the Agomelatine crystalline forms which were disclosed in the above mentioned publications, except for Form II as disclosed in EP1564202, were found to be unstable during storage, whereupon they transformed into Form II, or they found to be not reproducible.

The Agomelatine Urea complex of the present invention is reproducible and also stable during storage. For example, at 25°C / 60% relative humidity, or at 30°C / 65% relative humidity, or at 40°C / 75% relative humidity, the Agomelatine-urea complex of the present invention remained stable for at least 162 days.

In addition, the Agomelatine-urea complex exhibits significantly enhanced solubility and enhanced rate of dissolution, compared to the moderately soluble Agomelatine Form II. The enhanced solubility, which is apparently facilitated by the stoichoimetric incorporation of the highly soluble urea into the Agomelatine crystal lattice, constitutes an important advantage, in particular in sublingual formulations.

The Agomelatine-urea complex may also improve stability and shelf-life of the API, or give rise to improved processing or handling properties during formulation. The complex may also provide improvements for the final dosage form, for instance by improving the dissolution profile, or bioavailability of the finished product, or by improving the shelf life of the finished dosage form.

In light of the above, Agomelatine urea might have superior alternative to Agomelatine Form II.

In one aspect, the present invention provides an Agomelatine-urea complex.

The Agomelatine-urea complex of the present invention can be present in isolated form or be in a solution. Preferably, the urea and agomelatine molecules are present in the complex at an about equimolar ratio (i.e. a 1:1 stoichiometry of urea and agomelatine). In other embodiments, excess urea may be present in the Agomelatine-urea complex

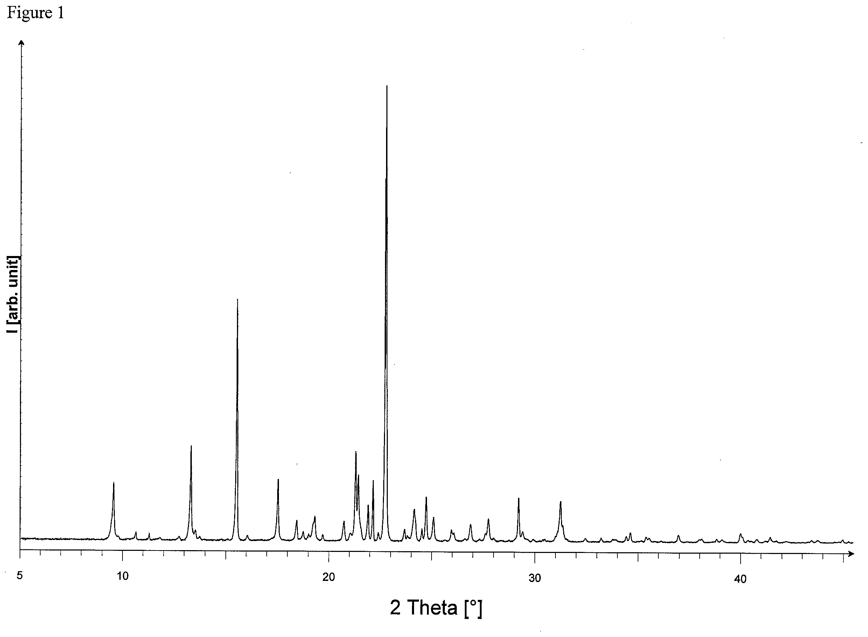

The present invention also provides a crystalline form of the Agomelatine-urea complex. A person skilled in the art would be able to characterize the above crystalline form by identifying one or more characteristic peaks in a powder X-ray diffraction analysis of that form. For example, the skilled person would be able to characterize the above form by selecting one or more characteristic peaks in the diffractogram provided in Figure 1.

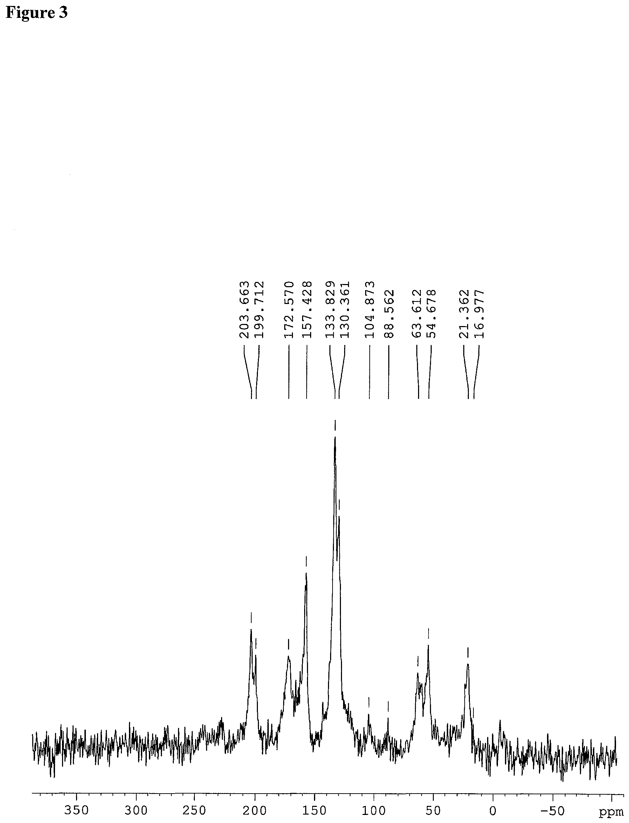

Accordingly, the crystalline form of the Agomelatine-urea complex is characterized by data selected from: a powder XRD pattern with peaks at 9.52°, 13.28°, 15.51°, 21.30°, and 22.75° 2θ ±0.2° 2θ; a powder XRD pattern substantially as depicted in Figure 1; a solid state ¹³C NMR spectrum with peaks at 21.4, 54.7, 130.4, 133.8, 157.4, 172.6, 199.7, and 203.7 ppm; a ¹³C NMR spectrum substantially as depicted in Figure 3; and any combinations thereof.

Alternatively, the crystalline form of the Agomelatine-urea complex is characterized by a powder XRD pattern with peaks at 9.52°, 13.28°, 15.51°, 21.30°, and 22.75° 2θ ±0.2° 2θ; or a powder XRD pattern substantially as depicted in Figure 1; or a solid state ¹³C NMR spectrum with peaks at 21.4, 54.7, 130.4, 133.8, 157.4, 172.6, 199.7, and 203.7 ppm; or a ¹³C NMR spectrum substantially as depicted in Figure 3; or any combinations thereof.

Alternatively, the crystalline form of the Agomelatine-urea complex can be characterized by a powder XRD pattern with peaks at 9.52°, 13.28°, 15.51 °, 21.30°, and 22.75° 2θ ±0.2° 2θ, and one or more additional peak(s) selected from the following peaks: 18.41°, 19.28°, 20.72°, 27.74°, and 31.26° 2θ ±0.2° 2θ.

The crystalline Agomelatine-urea complex can further be characterized by a single crystal X-ray structure having a monoclinic unit cell; symmetry space group P2₁/n having the parameters at 293 K approximately equal to the following: a=9.709(15) Å, b= 9.263(13) Å, c= 18.71(3) Å, alpha = 90°, beta = 101.79(2)°, gamma = 90°; and Volume = 1647(4) Å³.

In one embodiment, the crystalline Agomelatine-urea complex is characterized by the following atomic coordinates in the above-defined unit cell (see my comments below).

| Atom | *x* (x 10⁴) | *y* (x 10⁴) | *z* (x 10⁴) | *U*_{eq} (Å² x 10³) |
|---|---|---|---|---|
| C(1) | 7007(3) | 571(3) | 554(1) | 55(1) |
| C(2) | 9683(3) | 2610(3) | -1767(1) | 63(1) |
| C(3) | 10544(3) | 2622(3) | -2291(2) | 76(1) |
| C(4) | 10767(3) | 3861(4) | -2624(2) | 78(1) |
| C(5) | 10153(3) | 5184(3) | -2472(1) | 65(1) |
| C(6) | 9273(3) | 5182(3) | -1941(1) | 54(1) |
| C(7) | 9067(3) | 3858(3) | -1592(1) | 57(1) |
| C(8) | 8617(3) | 6495(3) | -1793(1) | 55(1) |
| C(9) | 8871(3) | 7724(3) | -2156(1) | 67(1) |
| C(10) | 9766(4) | 7734(4) | -2660(2) | 81(1) |
| C(11) | 10389(4) | 6485(4) | -2817(2) | 78(1) |
| C(12) | 8546(4) | 1183(3) | -995(2) | 83(1) |
| C(13) | 7681(3) | 6555(3) | -1239(1) | 58(1) |
| C(14) | 8547(3) | 6681(3) | -461(1) | 55(1) |
| C(15) | 7343(3) | 5349(3) | 353(1) | 56(1) |
| C(16) | 6462(3) | 5425(3) | 928(2) | 78(1) |
| N(1) | 8254(3) | 1065(2) | 917(1) | 79(1) |
| N(2) | 6145(3) | 1537(2) | 171(1) | 84(1) |
| N(3) | 7701(2) | 6597(2) | 99(1) | 57(1) |
| O(1) | 6684(2) | -725(2) | 586(1) | 66(1) |
| O(2) | 9515(2) | 1286(2) | -1471(1) | 79(1) |
| O(3) | 7698(2) | 4177(2) | 124(1) | 74(1) |
| H(1A) | 8843 | 485 | 1177 | 95 |
| H(1B) | 8463 | 1963 | 890 | 95 |
| H(2A) | 5330 | 1274 | -67 | 101 |
| H(2B) | 6403 | 2424 | 161 | 101 |
| H(3A) | 7427 | 7385 | 270 | 68 |
| H(3B) | 10958 | 1770 | -2406 | 92 |
| H(4A) | 11341 | 3849 | -2965 | 93 |
| H(7A) | 8511 | 3839 | -1242 | 69 |
| H(9A) | 8437 | 8581 | -2065 | 81 |
| H(10A) | 9932 | 8591 | -2887 | 97 |
| H(11A) | 10972 | 6493 | -3154 | 93 |
| H(12A) | 8523 | 208 | -825 | 124 |
| H(12B) | 7625 | 1457 | -1254 | 124 |
| H(12C) | 8838 | 1816 | -586 | 124 |
| H(13A) | 7108 | 5690 | -1279 | 70 |
| H(13B) | 7055 | 7379 | -1343 | 70 |
| H(14A) | 9243 | 5915 | -380 | 65 |
| H(14B) | 9048 | 7594 | -413 | 65 |
| H(16A) | 6262 | 4465 | 1072 | 117 |
| H(16B) | 5595 | 5916 | 734 | 117 |
| H(16C) | 6967 | 5941 | 1345 | 117 |

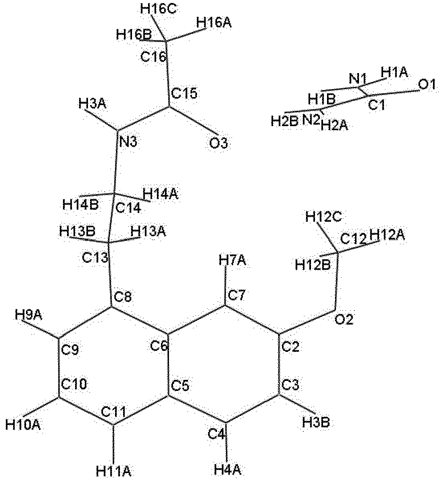

The Agomelatine-urea complex can be prepared, for example, by a process comprising co-milling Agomelatine with urea until the molecular complex of Agomelatine and urea is formed. The crystalline Agomelatine-urea complex may also be prepared by slow evaporation of a saturated solution of the Agomelatine-urea complex in a suitable solvent, such as ethanol, at ambient temperature.

The above mentioned Agomelatine-urea complex can *inter alia* be used to prepare Agomelatine polymorphs, for example, Agomelatine Form II, as described in EP1564202, or it can be used to prepare other complexes of Agomelatine.

In addition, the Agomelatine-urea complex can also be used to prepare pharmaceutical compositions. Hence, the present invention further encompasses 1) a pharmaceutical composition comprising the Agomelatine-urea complex or crystalline form thereof, as described above, and at least one pharmaceutically acceptable excipient; and 2) a process for preparing the pharmaceutical composition comprising combining the above mentioned Agomelatine-urea complex or the particular solid state form thereof, and at least one acceptable excipient.

The pharmaceutical composition can be used as a medicament, for example for treating depression.

The invention is further defined by reference to the following examples which should not be construed as limiting the scope of the present invention. The examples describe in detail the preparation of the complex and the compositions and further illustrate the uses and processes involving the Agomelatine-urea complex as described herein.

### EXAMPLES

### PXRD method

For powder XRD characterization, the samples were, if necessary, ground in an agate mortar and prepared on a flat specimen holder made from a silicon single crystal. Measurements were performed on a Bruker AXS D8 Advance powder X-ray diffractometer (Bruker AXS, Karlsruhe, Germany). The specimen holder was rotated in the z0 plane at 20 rpm during measurement. The measurement conditions were as follows: Radiation CuK_{α1} of wavelength 1.54184 Å, Source 40 kV / 40 mA, detector: PSD-LynxEye Range 4° 2θ; monochromator: Johansson Germanium Single Crystal; scan range of 3° - 50° 2θ; step width of 0.00922° 2θ; and measuring time of 10 sec/step. Raw data were evaluated using the program EVA (Bruker-AXS, Karlsruhe, Germany).

### Solid state NMR method

The ¹³C CPMAS NMR spectra were recorded at transmitter frequencies of 400.13 MHz and 100.639 MHz for ¹H and ¹³C on a Bruker ASX400 NMR spectrometer. A standard 7 mm Bruker MAS probe was used at rotation frequencies of 7 kHz. The 90° pulse length for ¹H was 9.0 µs (ωrf(¹H) = ωrf(¹³C) = 28 kHz). The recycle delay was 30s. A total of 800 scans were accumulated and tetramethylsilane was used as reference for both ¹H and ¹³C.

### Example 1: Preparing of Agomelatine-urea complex

Agomelatine (Form II) and urea (0.308 mmol) were placed in a steel crucible containing a grinding ball (steel, d = 10 mm). The crucible was mounted on a Mini-Mill PULVERISETTE 23 (Fritsch) and the mixture was ground for 30 minutes at an oscillation rate of 50 Hz. The Agomelatine-urea co-crystal that was produced was colorless to slightly ocher, and was a fine crystalline powder. The product may be recrystallized by slow evaporation of a saturated solution of the Agomelatine-urea co-crystal in ethanol at ambient temperature.

### Example 2: Preparation of Agomelatine-Urea-cocrystals with seeding

Agomelatine (7.0 g, 28.8 mmol) and urea (1.75 g, 29.1 mmol) were dissolved in ethanol (20mL) at reflux temperature.
The solution was filtered hot through a folded filter into a flask
containing a small amount of seed crystals (Agomelatine-Urea-cocrystals, obtainable for example by the procedure of Example 1). Immediate crystallization occurred, a small part of the product already formed in the filter. After 2 hours at room temperature the crystallized solid was isolated by vacuum filtration, washed with a small amount of cold ethanol and dried at 35°C / 20 mbar overnight. A colourless coarse crystalline material was obtained (6.13 g, 70%), whose identity was confirmed by powder XRD, FT-IR, and DSC analysis.

### Example 3: Preparing Form II

Sodium acetate (purity 98.5%, 35.32 g, 0.424 mol) and 2-(7-methoxy-1-naphthyl)-ethylamine hydrochloride (91.64 g, 0.385 mol) were suspended in ethanol (175 mL) at reflux temperature. Acetic acid anhydride (purity 98.5%, 39.21 mL, 0.409 mol) was added dropwise with stirring. The mixture was then refluxed for 40 minutes. After addition of water (326 mL), the mixture was cooled to room temperature and stirred for 14 hours. Agomelatine precipitated as polymorph II (86.16 g, 92%).

### Example 4: Single crystal X-ray diffraction

A single crystal of the Agomelatine-urea complex, which was suitable for X-ray diffraction measurement, was obtained by slow evaporation of a solution of the Agomelatine-urea co-crystal in ethanol at room temperature. The measurement was performed on a Rigaku XtaLab mini diffractometer at 293K. Crystallographic data are given below.

| | |
|---|---|
| Empirical formula | C₁₆H₂₁N₃O₃ |
| Formula weight | 303.36 |
| Wavelength | 0.71075 Å |
| Crystal system, space group | monoclinic, P2₁/n |
| Unit cell dimensions | a = 9.709(15) Å |
| | b = 9.263(13) Å |
| | c = 18.71(3) Å |
| | alpha = 90° |
| | beta = 101.79(2)° |
| | gamma = 90° |
| Volume | 1647(4) Å³ |
| Z, Calculated density | 4, 1.223 g/cm³ |
| Absorption coefficient | 0.086 mm⁻¹ |
| F(000) | 648 |
| Crystal size | 0.2 x 0.2 x 0.2 mm |
| Measurement temperature | 293 K |
| Theta range for data collection | 2.22 to 25.00 deg. |
| Limiting indices | -11<=h<=7; |
| | -11<=k<=8; |
| | -22<=l<=19 |
| Reflections collected / unique | 5729 / 2880 [R(int) = 0.03001 |
| Completeness to theta = 25.00 | 99.5 % |
| Refinement method | Full-matrix least-squares on F² |
| Data / restraints / parameters | 2880 / 0 / 199 |
| Goodness-of-fit on F² | 1.087 |
| Final R indices [I>2sigma(I)] | R₁ = 0.0622, wR₂ = 0.1454 |
| R indices (all data) | R₁ = 0.0980, wR₂ = 0.1692 |
| Largest diff. peak and hole | 0.230 and -0.145 eÅ⁻³ |

## Claims

1. Agomelatine-urea complex.

2. The Agomelatine-urea complex of claim 1, which is isolated.

3. The Agomelatine-urea complex of claim 1 or claim 2, wherein the urea and agomelatine molecules are present at an equimolar ratio (1:1).

4. A crystalline form of the Agomelatine-urea complex according to any one of claims 1 to 3, **characterized by** data selected from: a powder XRD pattern with peaks at 9.52°, 13.28°, 15.51°, 21.30°, and 22.75° 2θ ±0.2° 2θ; a powder XRD pattern substantially as depicted in Figure 1; a solid state ¹³C NMR spectrum with peaks at 21.4, 54.7, 130.4, 133.8, 157.4, 172.6, 199.7, and 203.7 ppm; a ¹³C NMR spectrum substantially as depicted in Figure 3; and any combinations thereof.

5. The crystalline form of the Agomelatine-urea complex according to any one of claims 1 to 3, **characterized by** a powder XRD pattern with peaks at 9.52°, 13.28°, 15.51°, 21.30°, and 22.75° 2θ ±0.2° 2θ.

6. The crystalline form of the Agomelatine-urea complex according to any one of claims 1 to 3, **characterized by** a powder XRD pattern substantially as depicted in Figure 1.

7. The crystalline form of the Agomelatine-urea complex according to any one of claims 1 to 3, **characterized by** a solid state ¹³C NMR spectrum with peaks at 21.4, 54.7, 130.4, 133.8, 157.4, 172.6, 199.7, and 203.7 ppm.

8. The crystalline form of the Agomelatine-urea complex according to any one of claims 1 to 3, **characterized by** a ¹³C NMR spectrum substantially as depicted in Figure 3.

9. Use of Agomelatine-urea complex according to any one of claims 1 to 8 for the preparation of Agomelatine.

10. A pharmaceutical composition comprising the Agomelatine-urea complex according to any one of claims 1 to 8, and at least one pharmaceutically acceptable excipient.

11. Use of the Agomelatine-urea complex according to any one of claims 1 to 8 for the manufacture of a medicament.

12. A process for preparing a pharmaceutical composition comprising combining the Agomelatine-urea complex according to any one of claims 1 to 8 and at least one pharmaceutically acceptable excipient.

13. The Agomelatine-urea complex according to any one of claims 1 to 8 or the pharmaceutical composition of claim 10 for use as a medicament.

14. The Agomelatine-urea complex according to any one of claims I to 8 or the pharmaceutical composition of claim 10 for use in the treatment of depression.

15. The Agomelatine-urea complex according to any one of claims 1 to 3, or a crystalline form thereof according to any one of claims 4 to 8, or a pharmaceutical composition according to claim 10, for use in the treatment of depression.

## Patentansprüche

1. Agomelatin-Harnstoff-Komplex.

2. Agomelatin-Harnstoff-Komplex gemäß Anspruch 1, der isoliert ist.

3. Agomelatin-Harnstoff-Komplex gemäß Anspruch 1 oder Anspruch 2, wobei die Harnstoff- und Agomelatin-Moleküle in einem äquimolaren Verhältnis (1:1) vorhanden sind.

4. Kristalline Form des Agomelatin-Harnstoff-Komplexes gemäß einem der Ansprüche 1 bis 3, **gekennzeichnet durch** Daten ausgewählt aus: einem Pulver-XRD-Muster mit Maxima bei 9,52°, 13,28°, 15,51°, 21,30° und 22,75° 2θ ± 0,2° 2θ; einem Pulver-XRD-Muster im Wesentlichen wie in Figur 1 dargestellt; einem Festkörper-¹³C-NMR-Spektrum mit Maxima bei 21,4, 54,7, 130,4, 133,8, 157,4, 172,6, 199,7 und 203,7 ppm; einem ¹³C-NMR-Spektrum im Wesentlichen wie in Figur 3 dargestellt; und beliebigen Kombinationen davon.

5. Kristalline Form des Agomelatin-Harnstoff-Komplexes gemäß einem der Ansprüche 1 bis 3, **gekennzeichnet durch** ein Pulver-XRD-Muster mit Maxima bei 9,52°, 13,28°, 15,51°, 21,30° und 22,75° 2θ ± 0,2° 2θ.

6. Kristalline Form des Agomelatin-Harnstoff-Komplexes gemäß einem der Ansprüche 1 bis 3, **gekennzeichnet durch** ein Pulver-XRD-Muster im Wesentlichen wie in Figur 1 dargestellt.

7. Kristalline Form des Agomelatin-Harnstoff-Komplexes gemäß einem der Ansprüche 1 bis 3, **gekennzeichnet durch** ein Festkörper-¹³C-NMR-Spektrum mit Maxima bei 21,4, 54,7, 130,4, 133,8, 157,4, 172,6, 199,7 und 203,7 ppm.

8. Kristalline Form des Agomelatin-Harnstoff-Komplexes gemäß einem der Ansprüche 1 bis 3, **gekennzeichnet durch** ein ¹³C-NMR-Spektrum im Wesentlichen wie in Figur 3 dargestellt.

9. Verwendung des Agomelatin-Harnstoff-Komplexes gemäß einem der Ansprüche 1 bis 8 für die Herstellung von Agomelatin.

10. Pharmazeutische Zusammensetzung, umfassend den Agomelatin-Harnstoff-Komplex gemäß einem der Ansprüche 1 bis 8 und wenigstens einen pharmazeutisch verträglichen Hilfsstoff.

11. Verwendung des Agomelatin-Harnstoff-Komplexes gemäß einem der Ansprüche 1 bis 8 für die Herstellung eines Medikaments.

12. Verfahren zum Herstellen einer pharmazeutischen Zusammensetzung, umfassend den Agomelatin-Harnstoff-Komplex gemäß einem der Ansprüche 1 bis 8 und wenigstens einen pharmazeutisch verträglichen Hilfsstoff.

13. Agomelatin-Harnstoff-Komplex gemäß einem der Ansprüche 1 bis 8 oder pharmazeutische Zusammensetzung gemäß Anspruch 10 für die Verwendung als Medikament.

14. Agomelatin-Harnstoff-Komplex gemäß einem der Ansprüche 1 bis 8 oder pharmazeutische Zusammensetzung gemäß Anspruch 10 für die Verwendung bei der Behandlung von Depression.

15. Agomelatin-Harnstoff-Komplex gemäß einem der Ansprüche 1 bis 3 oder kristalline Form davon gemäß einem der Ansprüche 4 bis 8 oder pharmazeutische Zusammensetzung gemäß Anspruch 10 für die Verwendung bei der Behandlung von Depression.

## Revendications

1. Complexe agomélatine-urée.

2. Complexe agomélatine-urée selon la revendication 1, qui est isolé.

3. Complexe agomélatine-urée selon la revendication 1 ou la revendication 2, où les molécules d'urée et d'agomélatine sont présentes dans un rapport équimolaire (1:1).

4. Forme cristalline du complexe agomélatine-urée selon l'une quelconque des revendications 1 à 3, **caractérisée par** des données choisies parmi les suivantes : un diagramme de diffraction X de poudre avec des pics à 9,52°, 13,28°, 15,51°, 21,30° et 22,75° 2θ ± 0,2° 2θ ; un diagramme de diffraction X de poudre substantiellement tel qu'illustré par la Figure 1 ; un spectre de RMN ¹³C du solide avec des pics à 21.4, 54.7, 130.4, 133.8, 157.4, 172.6, 199.7 et 203.7 ppm ; un spectre de RMN ¹³C substantiellement tel qu'illustré par la Figure 3 ; n'importe laquelle de leurs combinaisons.

5. Forme cristalline du complexe agomélatine-urée selon l'une quelconque des revendications 1 à 3, **caractérisée par** un diagramme de diffraction X de poudre avec des pics à 9,52°, 13,28°, 15,51°, 21,30° et 22,75° 2θ ± 0,2° 2θ.

6. Forme cristalline du complexe agomélatine-urée selon l'une quelconque des revendications 1 à 3, **caractérisée par** un diagramme de diffraction X de poudre substantiellement tel qu'illustré par la Figure 1.

7. Forme cristalline du complexe agomélatine-urée selon l'une quelconque des revendications 1 à 3, **caractérisée par** un spectre de RMN ¹³C du solide avec des pics à 21.4, 54.7, 130.4, 133.8, 157.4, 172.6, 199.7 et 203.7 ppm.

8. Forme cristalline du complexe agomélatine-urée selon l'une quelconque des revendications 1 à 3, **caractérisée par** un spectre de RMN ¹³C substantiellement tel qu'illustré par la Figure 3.

9. Utilisation du complexe agomélatine-urée selon l'une quelconque des revendications 1 à 8 dans la préparation d'agomélatine.

10. Composition pharmaceutique comprenant le complexe agomélatine-urée selon l'une quelconque des revendications 1 à 8, et au moins un excipient pharmaceutiquement acceptable.

11. Utilisation du complexe agomélatine-urée selon l'une quelconque des revendications 1 à 8 dans la fabrication d'un médicament.

12. Procédé élaboration d'une composition pharmaceutique comprenant la combinaison du complexe agomélatine-urée selon l'une quelconque des revendications 1 à 8, et au moins un excipient pharmaceutiquement acceptable.

13. Complexe agomélatine-urée selon l'une quelconque des revendications 1 à 8 ou composition pharmaceutique selon la revendication 10, pour utilisation en tant que médicament.

14. Complexe agomélatine-urée selon l'une quelconque des revendications 1 à 8 ou composition pharmaceutique selon la revendication 10, pour utilisation dans le traitement de la dépression.

15. Complexe agomélatine-urée selon l'une quelconque des revendications 1 à 3, ou forme cristalline de celui-ci selon l'une quelconque des revendications 4 à 8, ou composition pharmaceutique selon la revendication 10, pour utilisation dans le traitement de la dépression.
